(19) Europäisches Patentamt
European Patent Office
Office européen des brevets

(11) **EP 3 788 946 B1**

(12) **EUROPÄISCHE PATENTSCHRIFT**

(45) Veröffentlichungstag und Bekanntmachung des Hinweises auf die Patenterteilung:
**13.05.2026 Patentblatt 2026/20**

(21) Anmeldenummer: **20192007.1**

(22) Anmeldetag: **20.08.2020**

(51) Internationale Patentklassifikation (IPC):
*A61B 1/00* (2006.01)    *A61B 1/012* (2006.01)
*A61B 1/05* (2006.01)    *A61B 1/12* (2006.01)
*F28D 15/02* (2006.01)    *A61B 1/06* (2006.01)

(52) Gemeinsame Patentklassifikation (CPC):
**A61B 1/0008; A61B 1/128;** A61B 1/00193;
A61B 1/012; A61B 1/05; A61B 1/0676

(54) **VORRICHTUNG ZUR WÄRMEABLEITUNG UND VERWENDUNG EINER SOLCHEN VORRICHTUNG**

DEVICE FOR HEAT DISSIPATION AND USE OF SUCH A DEVICE

DISPOSITIF DE DISSIPATION THERMIQUE ET UTILISATION D'UN TEL DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2019 DE 102019123908**

(43) Veröffentlichungstag der Anmeldung:
**10.03.2021 Patentblatt 2021/10**

(73) Patentinhaber: **Karl Storz SE & Co. KG
78532 Tuttlingen (DE)**

(72) Erfinder:
• **HENI, Andreas
78532 Tuttlingen (DE)**
• **KUPFERSCHMID, Markus
78532 Tuttlingen (DE)**
• **ULMSCHNEIDER, Daniel
78532 Tuttlingen (DE)**
• **FORSTER, Jonas
78532 Tuttlingen (DE)**

(56) Entgegenhaltungen:
DE-A1- 102010 024 003    DE-A1- 102014 107 205
DE-B3- 102017 118 941    US-A- 3 391 728
US-A1- 2017 258 309

**Beschreibung**

Die vorliegende Erfindung betrifft eine Vorrichtung zur Wärmeableitung mit einer Wärmequelle, einer Wärmesenke und einem Wärmeleitelement. Die Erfindung betrifft ferner die Verwendung einer solchen Vorrichtung zur Wärmeableitung in einem Videoendoskop.

In der Medizintechnik stellt gerade bei chirurgischen Instrumenten die Überwachung und Kontrolle von Temperatur und Wärme, das sogenannte Thermomanagement, eine große Herausforderung dar. So wird auf engem Raum durch elektrische Bauteile und durch die notwendige Beleuchtung Verlustwärme erzeugt, die aufgrund der beengten Verhältnisse auch nur mit erheblichem konstruktiven Aufwand abgeführt werden kann.

US 2017/0258309 A1 offenbart ein endoskopisches Instrument mit einer wärmeerzeugenden Komponente, die über eine bewegliche Kontaktstruktur mit einem Kühlkörper thermisch gekoppelt ist. Die bewegliche Kontaktstruktur nimmt die Relativbewegung zwischen der Wärmeleitung und der Wärmesenke auf, um die Wärmekopplung zwischen diesen Elementen aufrechtzuerhalten und eine Beschädigung einer solchen Wärmekopplung zu verhindern, wenn das Instrument erhöhten Temperaturen ausgesetzt ist, beispielsweise während des Autoklavierens.

US 3,391,728 offenbart eine Wärmequelle und eine Wärmesenke, die durch flüssiges Metall voneinander getrennt sind, das in einem abgedichteten Gehäuse enthalten ist, das einen Faltenbalg enthält und die Wärmequelle und die Wärmesenke berührt, sowie Mittel, die auf die Temperatur der Wärmequelle ansprechen, um den Druck auf das flüssige Metall physikalisch zu steuern, wodurch ein reduzierter Druck bewirkt, dass der flüssige Wärmeleiter von der Grenzfläche zwischen der Wärmequelle und der Wärmesenke entfernt wird.

Es ist eine Aufgabe der vorliegenden Erfindung, eine Vorrichtung zur Wärmeableitung aufzuzeigen, die auch unter beengten räumlichen Verhältnissen eine zuverlässige Ausrichtung mehrerer elektronischer Bauteile zueinander gewährleistet. Ferner soll eine entsprechende Verwendung aufgezeigt werden.

Gemäß einem ersten Aspekt der Erfindung wird die Aufgabe gelöst durch eine Vorrichtung zur Wärmeableitung mit einer Wärmequelle, einer Wärmesenke und einem Wärmeleitelement, wobei das Wärmeleitelement entlang eines Wärmeleitwegs Wärmeenergie von der Wärmequelle zur Wärmesenke führt, und wobei das Wärmeleitelement derart an der Wärmequelle und der Wärmesenke angeordnet ist und sich mit steigender Temperatur des Wärmeleitelements derart physikalisch verändert, dass a) sich eine erste Querschnittsfläche zwischen der Wärmequelle und dem Wärmeleitelement und/oder eine zweite Querschnittsfläche zwischen dem Wärmeleitelement der Wärmesenke vergrößert, und/oder b) sich die Länge des Wärmeleitwegs verkürzt.

Im Zusammenhang mit der Erfindung haben die Erfinder erkannt, dass es bei der Ableitung von Wärme von zwei Bauelementen, die eine genaue Position zueinander haben sollen, besondere Herausforderungen gibt. So besteht bei der Wärmeabfuhr die Situation, dass mit bekannten Vorrichtungen zwar Wärme von beiden Bauteilen abgeleitet wird, es jedoch nicht sichergestellt ist, dass die Bauteile auf zumindest ungefähr derselben Temperatur bleiben. Eine solche Temperaturdifferenz kann beispielsweise dadurch entstehen, dass sich der Wärmestrom von dem ersten Bauteil als Wärmequelle zu einer entsprechenden Wärmesenke von dem Wärmestrom von dem zweiten Bauteil zur Wärmesenke unterscheidet. Außerdem besteht die Möglichkeit, dass sich die beiden Bauteile, selbst wenn sie baugleich sind, unterschiedlich erwärmen. So ergibt sich gerade bei mehrkanaligen Endoskopen oder Exoskopen eine neue Herausforderung, bei denen es auch auf die optimale Ausrichtung und Justage zweier optischer Kanäle zueinander ankommt.

Eine Besonderheit der Erfindung liegt darin, dass eine Regelung der Wärmeableitung durch einen passiven Aufbau erzielt wird. Es wird hier das physikalische Prinzip ausgenutzt, dass der Wärmefluss zwischen einer Wärmequelle und einer Wärmesenke umso größer ist, desto größer die Querschnittsfläche A des Wärmeleitwegs zwischen der Wärmequelle mit der Temperatur $T_1$ und der Wärmesenke mit der Temperatur $T_2$ ist und/oder desto geringer die Länge d des Wärmeleitwegs ist. Dieser physikalische Hintergrund wird mit der folgenden Formel beschrieben:

$$\dot{Q} = \alpha \cdot \frac{A}{d} \cdot (T_1 - T_2)$$

In diesem Zusammenhang soll die Querschnittsfläche insbesondere als die wirksame Querschnittsfläche verstanden werden und die Länge insbesondere als die wirksame Länge verstanden werden. Dies bedeutet, dass die Querschnittsfläche und/oder die Länge betrachtet werden soll, die den Wärmefluss entsprechend der oben genannten Formel beeinflusst

Da die Vorrichtung aus lediglich passiven Elementen realisiert werden kann, kann sie gerade bei beengten Platzverhältnissen eingesetzt werden. Zudem ermöglicht das Fehlen von aktiven Elementen einen besonders langen störungsfreien Betrieb. Es ist selbstverständlich möglich, die aufgezeigte Vorrichtung auch mit zusätzlichen aktiven Komponenten für eine Überwachung und Steuerung, wie z.B. einem Temperatursensor oder einer Temperaturregelung der Wärmesenke, zu versehen. Es wird aber für bestimmte Anwendungsfälle als vorteilhaft angesehen, dass allein der passive Aufbau eine Temperaturregelung bewirken kann.

Die Temperaturregelung funktioniert prinzipiell wie folgt. Steigt die Temperatur an der Wärmequelle an, so erwärmt sich auch das Wärmeleitelement. Die physikalischen Eigenschaften des Wärmeleitelements, einschließlich seiner räumlichen Ausgestaltung, sind nun derart gewählt, dass das Wärmeleitelement durch seine physikalische Veränderung die Querschnittsfläche des Wärmeleitpfads vergrößert oder die effektive Länge des Wärmeleitpfads verkürzt wird. Die Erhöhung des Querschnitts und/oder die Verkürzung der Länge des Wärmeleitpfads führen zu einem erhöhten Wärmestrom von der Wärmequelle zur Wärmesenke. Dadurch wird ein weiteres Erwärmen der Wärmequelle gebremst, verhindert oder in eine Abkühlung der Wärmequelle umgekehrt.

Wenn sich die Wärmequelle abkühlt, kühlt sich auch das Wärmeleitelement ab. Aufgrund seiner physikalischen Veränderung verringert sich nun der Querschnitt des Wärmeleitpfads und/oder vergrößert sich die Länge des Wärmeleitpfads. Dadurch wird der Wärmestrom von der Wärmequelle zur Wärmesenke verringert. Auf diese Weise kann ein weiteres Abkühlen der Wärmequelle gebremst, verhindert oder in eine Erwärmung umgekehrt werden.

Idealerweise werden die verwendeten Materialien und die physikalischen Abmessung derart gewählt, dass das Wärmeleitelement bereits auf geringe Temperaturschwankungen reagiert. Dadurch ist es möglich, dass die Temperatur der Wärmequelle zumindest ungefähr konstant gehalten werden kann oder innerhalb eines vorgegebenen Toleranzbereichs gehalten werden kann. Da ein weiteres Bauelement prinzipiell mit derselben Vorrichtung gekühlt werden kann, findet die Wärmeableitung an einem ersten Bauelement zwar prinzipiell unabhängig von einer Kühlung des zweiten Bauelements statt, doch bewirkt die Vorrichtung, dass beide Bauelemente auf zumindest ungefähr dieselbe Temperatur gekühlt werden.

Bei einigen Ausgestaltungen kann durch gezielte Nutzung von Dehnungen aufgrund von Temperaturänderungen eine Temperaturregelung realisiert werden. Diese Temperaturregelung hält die Temperatur in Bauteilen, die eine genaue Positionierung erfordern, auf einer bestimmten Temperatur oder innerhalb eines bestimmten Temperaturbereichs., Somit können Längenänderungen durch Temperaturänderungen reduziert oder verhindert werden, die die Positionierung oder die Einstellung dieser Bauteile verändern könnten.

Bei einigen Ausgestaltungen liegt das Wärmeleitelement flächig an der Wärmequelle und/oder Wärmesenke an. Bevorzugt liegt dabei das Wärmeleitelement mit einer Fläche an einer Fläche der Wärmequelle an. Insbesondere ist die Fläche des Wärmeleitelements dann gleitend über der Fläche der Wärmequelle angeordnet, wobei die beiden Flächen auch bei Temperaturänderungen in physikalischem Kontakt bleiben.

Damit ist die Aufgabe vollständig gelöst.

Bei einer bevorzugten Ausgestaltung weist das Wärmeleitelement zur Steigerung der Wärmeableitung eine Heatpipe auf oder wird von einem Fluid durchströmt.

Mit dieser Ausgestaltung kann der Wärmestrom von der Wärmequelle zur Wärmesenke vergrößert werden.

Bei einer weiteren vorteilhaften Ausgestaltung weist die Wärmequelle eine erste Ausnehmung auf, in der ein erster Abschnitt des Wärmeleitelements angeordnet ist, oder weist das Wärmeleitelement eine erste Ausnehmung auf, in der ein erster Abschnitt der Wärmequelle angeordnet ist.

Diese Ausgestaltung ermöglicht es auf konstruktiv einfache Weise, die Wärmequelle und das Wärmeleitelement so in physikalischem Kontakt miteinander anzuordnen, dass dieser Kontakt auch bei Temperaturänderungen stets bestehen bleibt. Dabei erfolgt eine Ausdehnung des Wärmeleitelements bevorzugt in der Tiefe der Ausnehmung, wobei ein flächiger Kontakt zwischen dem Wärmeleitelement und der Wärmequelle an den Seiten der Ausnehmung fortwährend bestehen bleibt. Die erste Ausnehmung ist hier bevorzugt länglich ausgeführt, insbesondere in der Form eines Prismas, eines Zylinders oder eines Quaders.

Bei einer weiteren vorteilhaften Ausgestaltung weist die Wärmesenke eine zweite Ausnehmung auf, in der ein zweiter Abschnitt des Wärmeleitelements angeordnet ist, oder weist das Wärmeleitelement eine zweite Ausnehmung auf, in der ein zweiter Abschnitt der Wärmesenke angeordnet ist.

Wie zuvor bezüglich der ersten Ausnehmung im Zusammenspiel zwischen der Wärmequelle und dem Wärmeleitelement beschrieben, bietet auch diese Ausgestaltung eine gute Möglichkeit, den fortwährenden physikalischen Kontakt zwischen dem Wärmeleitelement und der Wärmesenke auch bei sich ändernden Temperaturen sicherzustellen.

Bei einer weiteren vorteilhaften Ausgestaltung ist die zweite Ausnehmung durch die Wärmesenke geführt und ist das Wärmeleitelement in der zweiten Ausnehmung durch die Wärmesenke geführt.

Bei dieser Ausgestaltung kann sich das Wärmeleitelement ebenfalls mit einer Fläche gleitend über die Innenfläche der Ausnehmung der Wärmesenke gleitend verlagern. Zusätzlich kann das Wärmeleitelement nun an seinem von der Wärmequelle abgewandten Ende an einem Widerlager befestigt sein.

Bei einer weiteren vorteilhaften Ausgestaltung sind die Wärmequelle, die Wärmesenke und das Wärmeleitelement entlang einer Geraden angeordnet, insbesondere entlang einer gemeinsamen Längsmittelachse.

Diese Ausgestaltung ermöglicht es auf einfache Weise, eine physikalische Wechselwirkung zwischen der Wärmequelle, der Wärmesenke und dem Wärmeleitelement herzustellen. Insbesondere kann dabei erreicht werden, dass eine Ausdehnung der Wärmequelle zu einer Verkürzung des Wärmeleitelements führt und/oder eine Verkürzung der Wärmequelle zu einer Ausdehnung des Wärmeleitelements führt.

Bei einer weiteren vorteilhaften Ausgestaltung sind die Wärmequelle, die Wärmesenke und das Wärmeleitelement innerhalb eines Gehäuses angeordnet, wobei eine dem Wärmeleitelement abgewandte Seite der Wärmequelle und/oder eine dem Wärmeleitelement abgewandte Seite der Wärmesenke am Gehäuse angeordnet ist.

Diese Ausgestaltung bietet ein Widerlager für die Wärmequelle und/oder die Wärmesenke. Auf diese Weise kann für die Wärmequelle und/oder die Wärmesenke ein ortsfester Punkt erzeugt werden. Daher kann insbesondere eine Auswirkung durch eine Längenänderung der Wärmequelle auf das Wärmeleitelement gut eingestellt werden.

Bei einer weiteren vorteilhaften Ausgestaltung ist an der dem Wärmeleitelement abgewandten Seite der Wärmequelle ein Bildsensor ausgebildet, der eine Blickachse aufweist, die durch eine Öffnung in einer Wand des Gehäuses aus dem Gehäuse heraus gerichtet ist. Diese Ausgestaltung ermöglicht es, dass der Bildsensor auf einer im Wesentlichen konstanten Temperatur oder innerhalb eines bestimmten Temperaturbereichs gehalten wird. Dadurch bleibt die Blickachse des Bildsensors im Wesentlichen konstant. Ferner, wenn ein zweiter Bildsensor einen Teil der Wärmequelle bildet oder ein zweiter Bildsensor eine zweite Wärmequelle einer zweiten derartigen Vorrichtung bildet, findet am ersten Bildsensor und am zweiten Bildsensor eine gleichartige Temperaturregelung statt. Damit können der erste Bildsensor und der zweite Bildsensor im Wesentlichen auf derselben Temperatur gehalten werden, selbst wenn sie unterschiedliche Wärmeleistungen abgeben.

Bei einer weiteren vorteilhaften Ausgestaltung weist die Vorrichtung ferner ein Steuerelement auf, das Wärmeenergie von der Wärmequelle aufnimmt und bei steigender Temperatur einen steigenden Druck auf das Wärmeleitelement ausübt.

Diese Ausgestaltung bietet die Möglichkeit, die Länge des Wärmeleitwegs zwischen der Wärmequelle und der Wärmesenke zu verkürzen. Dies erfolgt durch den Druck, der durch das Steuerelement ausgeübt wird und das Wärmeleitelement zusammendrückt. Durch die Verkürzung des Wärmeleitwegs erhöht sich der Wärmestrom. Auf diese Weise wird eine Erwärmung der Wärmequelle abgebremst, unterdrückt oder in eine Abkühlung umgedreht. Wenn sich die Wärmequelle abkühlt, kühlt sich auch das Steuerelement ab, und der Druck auf das Wärmeleitelement wird reduziert. Hierdurch expandiert das Wärmeleitelement in Richtung seiner ursprünglichen Form, so dass die Länge des Wärmeleitwegs nun vergrößert wird. Dies führt dazu, dass die Abkühlung der Wärmequelle gebremst, unterdrückt oder in eine Erwärmung umgekehrt wird.

Bei einer weiteren vorteilhaften Ausgestaltung weist die Vorrichtung einen Hebel mit einem ersten Hebelarm und einem zweiten Hebelarm auf, wobei das Steuerelement bei steigender Temperatur einen steigenden Druck auf den ersten Hebelarm ausübt, so dass der zweite Hebelarm über die Wärmesenke einen Druck auf das Wärmeleitelement ausübt.

Diese Ausgestaltung ermöglicht es, den Druck, den das Steuerelement indirekt auf das Wärmeleitelement ausübt, zu vergrößern. Dabei beträgt bei bevorzugten Ausgestaltungen das Verhältnis einer zweiten Länge des zweiten Hebelarms zu einer ersten Länge des ersten Hebelarms mindestens 1, bevorzugt mindestens 1,5, besonders bevorzugt mindestens 2 und insbesondere mindestens 2,5.

Bei einer weiteren vorteilhaften Ausgestaltung weist die Vorrichtung ferner ein Steuerelement auf, das Wärmeenergie von der Wärmequelle aufnimmt, und es bewegt sich zumindest ein Abschnitt des Steuerelements bei steigender Temperatur auf das Wärmeleitelement zu oder ein Druck auf das Wärmeleitelement wird erhöht.

Bei dieser Ausgestaltung wirkt das Steuerelement insbesondere direkt auf das Wärmeleitelement. Dadurch kann der Aufbau vereinfacht werden.

Bei einer weiteren vorteilhaften Ausgestaltung ist das Steuerelement als ein erster Streifen ausgebildet und weist ein Gegenelement auf, das als zweiter Streifen auf dem Steuerelement fest angeordnet ist, wobei das Gegenelement aus einem Material besteht, dass einen anderen Wärmeausdehnungskoeffizienten hat als das Steuerelement, wobei das Steuerelement mit dem Gegenelement so angeordnet ist, dass das Steuerelement bei steigender Temperatur mit steigendem Druck gegen das Wärmeleitelement drückt.

Bei dieser Ausgestaltung wird ein physikalisches Prinzip ähnlich einem Bi-Metall-Streifen ausgenutzt. Da das Gegenelement einen anderen Wärmeausdehnungskoeffizienten hat, insbesondere in dem Arbeitstemperaturbereich der Vorrichtung im Wesentlichen unverändert ist, führt die feste Verbindung zwischen Steuerelement und Gegenelement zu einer Verformung des Steuerelements, insbesondere zu einer Krümmung. Diese resultierende Kraft wird genutzt, um einen Druck gegen das Wärmeleitelement auszuüben. Durch den Druck wird das Wärmeleitelement zusammengedrückt, und der Wärmeleitweg verkürzt sich. Bei vorteilhaften Ausgestaltungen bilden das Steuerelement und das Gegenelement einen Bi-Metall-Streifen.

Bei einer weiteren vorteilhaften Ausgestaltung ist das Wärmeleitelement als Wärmeleitpad ausgebildet, dessen Dicke sich bei steigendem Druck durch das Steuerelement verringert.

Diese Ausgestaltung ist sehr robust. Das Wärmeleitpad kann bei steigendem Druck zusammengedrückt werden und expandiert bei nachlassendem Druck in Richtung seiner ursprünglichen Form.

Bei einer weiteren vorteilhaften Ausgestaltung weist das Wärmeleitelement ein erstes kammartiges Element und ein zweites kammartiges Element auf, die komplementär zueinander ausgebildet sind und miteinander kämmen, wobei das erste kammartige Element am Steuerelement angeordnet ist und das zweite kammartige Element an der Wärmesenke angeordnet ist, wobei sich das erste kammartige Element und das zweite kammartige Element bei steigendem Druck durch das Steuerelement weiter ineinanderschieben.

Bei dieser Ausgestaltung kann einerseits ausgenutzt werden, dass sich die Querschnittsfläche des Wärmeleitwegs vergrößert, je weiter die kammartigen Elemente ineinandergeschoben werden bzw. je weiter diese miteinander kämmen.

Gemäß einem weiteren Aspekt der Erfindung wird ein Videoendoskop mit einer Vorrichtung nach einem der vorhergehenden Ansprüche aufgezeigt, wobei die Wärmequelle einen Bildsensor aufweist. Bei einigen bevorzugten Ausgestaltungen ist die Wärmequelle als ein Bildsensor ausgebildet. Bei weiteren bevorzugten Ausgestaltungen ist die Wärmequelle als ein erster Bildsensor und ein zweiter Bildsensor ausgebildet oder weist die Wärmequelle einen ersten Bildsensor und einen zweiten Bildsensor auf. Bei anderen bevorzugten Ausgestaltungen des Videoendoskops weist das Videoendoskop zusätzlich zu der genannten ersten Vorrichtung eine zweite Vorrichtung auf, bei der die Wärmequelle als zweiter Bildsensor ausgebildet ist.

Gemäß einem dritten Aspekt wird die Verwendung einer zuvor beschriebenen Vorrichtung zur Wärmeableitung in einem Videoendoskop aufgezeigt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in den Zeichnungen dargestellt und wird in der nachfolgenden Beschreibung näher erläutert. Es zeigen:

Fig. 1    zeigt eine erste Ausführungsform einer Vorrichtung bei einer niedrigeren Temperatur;

Fig. 2    zeigt die erste Ausführungsform bei einer höheren Temperatur;

Fig. 3    zeigt eine zweite Ausführungsform einer Vorrichtung;

Fig. 4    zeigt eine dritte Ausführungsform einer Vorrichtung;

Fig. 5    zeigt eine vierte Ausführungsform einer Vorrichtung bei einer niedrigeren Temperatur;

Fig. 6    zeigt die vierte Ausführungsform bei einer höheren Temperatur;

Fig. 7    zeigt eine fünfte Ausführungsform einer Vorrichtung bei einer niedrigeren Temperatur;

Fig. 8    zeigt die fünfte Ausführungsform bei einer höheren Temperatur;

Fig. 9    zeigt eine sechste Ausführungsform einer Vorrichtung bei einer niedrigeren Temperatur;

Fig. 10    zeigt die fünfte Ausführungsform bei einer höheren Temperatur;

Fig. 11    zeigt eine erste Ausführungsform eines Videoendoskops;

Fig. 12    zeigt eine zweite Ausführungsform eines Videoendoskops; und

Fig. 13    zeigt eine dritte Ausführungsform eines Videoendoskops.

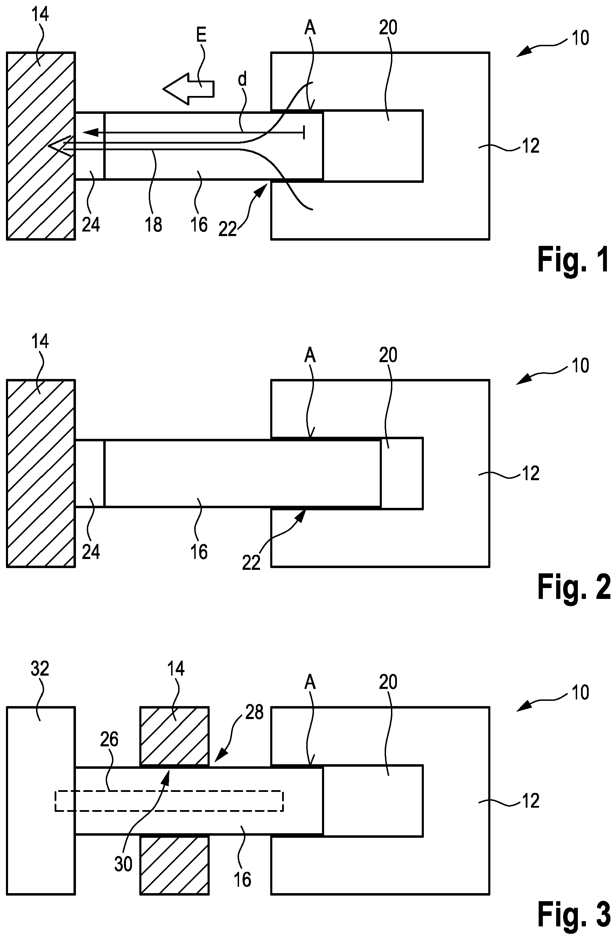

Fig. 1 zeigt eine erste Ausführungsform einer Vorrichtung 10 zur Wärmeableitung mit einer Wärmequelle 12, einer Wärmesenke 14 und einem Wärmeleitelement 16. Das Wärmeleitelement führt entlang eines Wärmeleitwegs 18. Wärmeenergie E von der Wärmequelle 12 zur Wärmesenke 14.

Das Wärmeleitelement 16 ist derart an der Wärmequelle 12 und der Wärmesenke 14 angeordnet und verändert sich mit steigender Temperatur des Wärmeleitelements 16 derart physikalisch, dass sich mindestens eine der zwei folgenden Veränderungen ergibt:

a) eine erste Querschnittsfläche A zwischen der Wärmequelle vergrößert sich und/oder eine zweite Querschnittsfläche zwischen dem Wärmeleitelement 16 und der Wärmesenke 14 vergrößert sich (hier nicht gezeigt);

b) eine Länge d des Wärmeleitwegs 18 verkürzt sich.

Die Wärmequelle 12 weist eine erste Ausnehmung 20 auf, in der ein erster Abschnitt 22 des Wärmeleitelements 16 angeordnet ist.

Bei dieser ersten Ausführungsform wird im Wesentlichen darauf abgestellt, dass sich die erste Querschnittsfläche A mit steigender Temperatur vergrößert. Bei dieser ersten Ausführungsform ist das Wärmeleitelement 16 über ein optionales Wärmeleitpad 24 mit der Wärmesenke 14 verbunden.

Fig. 2 zeigt die Situation, wenn sich bei der ersten Ausführungsform gemäß Fig. 1 die Temperatur der Wärmequelle 12 und damit auch die Temperatur des Wärmeleitelements 16 erhöht. Es ist zu erkennen, dass sich das Wärmeleitelement 16 in die Ausnehmung 20 der Wärmequelle 12 hinein verlängert hat. Dabei besteht weiterhin ein physikalischer flächiger Kontakt zwischen dem Wärmeleitelement 16 und der Wärmequelle 12.

Da sich nun die erste Querschnittsfläche A zwischen der Wärmequelle 12 und dem Wärmeleitelement 16 vergrößert hat, entsteht auch ein größerer Wärmestrom zwischen der Wärmequelle 12 und der Wärmesenke 14. Mit anderen Worten, die Wärmequelle 12 wird nun stärker gekühlt.

Fig. 3 zeigt eine zweite Ausführungsform der Vorrichtung 10. Hier und im Nachfolgenden werden bereits eingeführte Bezugszeichen für dieselben oder funktional gleichartige Elemente weiterverwendet und nicht erneut erläutert.

Bei der zweiten Ausführungsform weist das Wärmeleitelement 16 zur Steigerung der Wärmeableitung eine Heatpipe 26 auf. Alternativ kann das Wärmeleitelement 16 auch von einem Fluid durchströmt werden (nicht gezeigt). Die Wärmesenke 14 weist hier eine zweite Ausnehmung 28 auf, in der ein zweiter Abschnitt 30 des Wärmeleitelements 16 angeordnet ist. Konkret ist die zweite Ausnehmung 28 durch die Wärmesenke 14 geführt, und das Wärmeleitelement 16 ist in der zweiten Ausnehmung 28 durch die Wärmesenke 14 geführt.

Anders als bei der ersten Ausführungsform weist das Wärmeleitelement 16 hier kein Wärmeleitpad 24 auf. Stattdessen ist das Wärmeleitelement 16 hier an einem statischen Widerlager 32 angeordnet, welches auch bei einer Temperaturänderung bezüglich der Wärmequelle 12, der Wärmesenke 14 und dem Wärmeleitelement 16 als ortsunveränderlich verstanden werden soll.

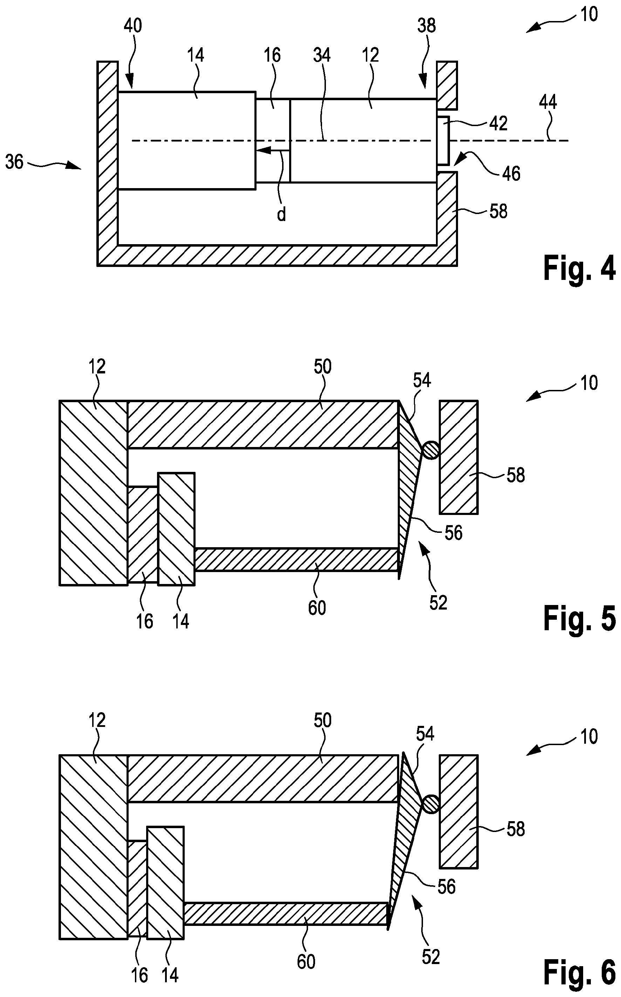

Fig. 4 zeigt eine dritte Ausführungsform der Vorrichtung 10, wobei hier die Wärmequelle 12, die Wärmesenke 14 und das Wärmeleitelement 16 entlang einer Geraden angeordnet sind, insbesondere entlang einer gemeinsamen Längsmittelachse 34. Die Wärmequelle 12, die Wärmesenke 14 und das Wärmeleitelement 16 sind hier innerhalb eines Gehäuses 36 angeordnet, wobei eine dem Wärmeleitelement 16 abgewandte Seite 38 der Wärmequelle 12 und/oder eine dem Wärmeleitelement 16 abgewandte Seite 40 der Wärmesenke 14 am Gehäuse 36 angeordnet ist.

Das Wärmeleitelement 16 ist hier als komprimierbares Wärmeleitpad ausgeführt. Wenn sich die Wärmequelle 12 erwärmt und ausdehnt, drückt die Wärmequelle 12 das Wärmeleitelement 16 zusammen und verkürzt so die Länge d des Wärmeleitwegs 18. Auf diese Weise erhöht sich der Wärmestrom von der Wärmequelle 12 zur Wärmesenke 14, so dass die Wärmequelle 12 mit steigender Temperatur stärker gekühlt wird. Wenn sich die Wärmequelle 12 abkühlt und schrumpft, expandiert das Wärmeleitelement 16 wieder und erhöht so die Länge d des Wärmeleitwegs 18. Auf diese Weise verringert sich der Wärmestrom von der Wärmequelle 12 zur Wärmesenke 14, so dass die Wärmequelle 12 mit sinkender Temperatur weniger gekühlt wird.

Bei dieser dritten Ausführungsform ist an der dem Wärmeelement 16 abgewandten Seite 38 der Wärmequelle 12 ein Bildsensor 42 ausgebildet. Der Bildsensor 42 weist eine Blickachse 44 auf, die durch eine Öffnung 46 in einer Wand 48 des Gehäuses 36 aus dem Gehäuse 36 herausgerichtet ist.

Fig. 5 zeigt eine vierte Ausführungsform, bei der die Vorrichtung 10 ferner ein Steuerelement 50 aufweist, das Wärmeenergie von der Wärmequelle 12 aufnimmt und bei steigender Temperatur einen steigenden Druck auf das Wärmeleitelement 16 ausübt. Hierdurch wird, wie bereits erläutert, bei steigender Temperatur das elastische Wärmeleitelement 16 zusammengedrückt und so die Länge d des Wärmeleitwegs 18 verkürzt.

Bei der vierten Ausführungsform wird hierfür ein Hebel 52 verwendet, der einen ersten Hebelarm 54 und einen zweiten Hebelarm 56 aufweist. Der Hebel 52 ist hier an einem Hebelwiderlager 58 angeordnet. Das Steuerelement 50 übt bei steigender Temperatur einen steigenden Druck auf den ersten Hebelarm 54 auf, so dass der zweite Hebelarm 56 über die Wärmesenke 14 einen Druck auf das Wärmeleitelement 16 ausübt. Die Übertragung des Drucks erfolgt hier beispielhaft über einen starren Stab 60.

Fig. 6 zeigt die Situation bei der vierten Ausführungsform, wenn sich die Wärmequelle 12 erwärmt hat und sich das Steuerelement 50 dadurch ausgedehnt hat. Es ist zu erkennen, dass das Steuerelement 50 gegen den ersten Hebelarm 54 gedrückt hat, wodurch der zweite Hebelarm 56 über den Stab 60 die Wärmesenke 14 in Richtung der Wärmequelle 12 gedrückt hat. Aufgrund des Drucks wurde das Wärmeleitelement 16 komprimiert, so dass sich die Länge d des Wärmeleitwegs 18 verkürzt hat.

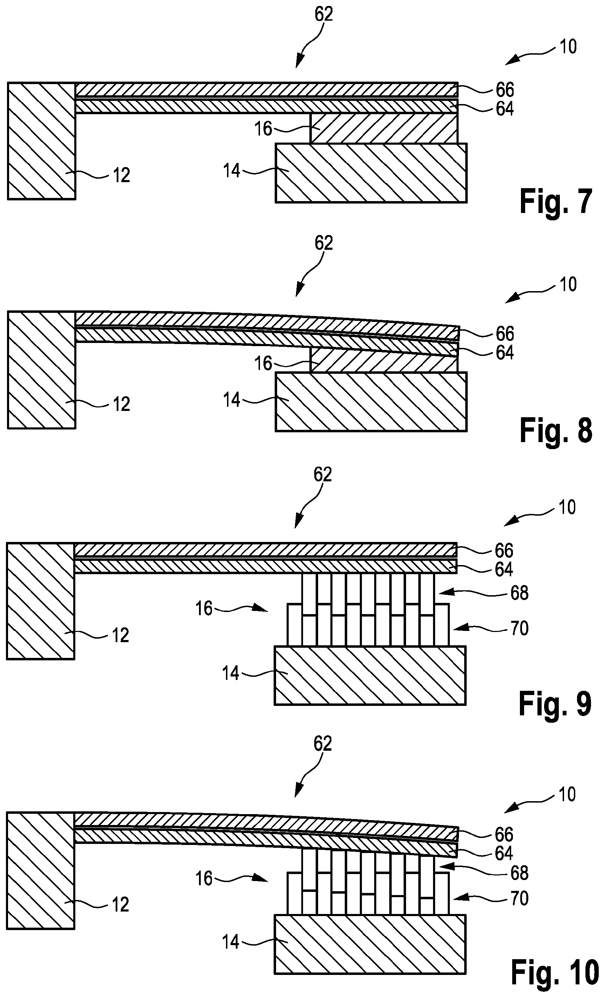

Fig. 7 zeigt eine fünfte Ausführungsform, bei der die Vorrichtung 10 ferner ein Steuerelement 62 aufweist, das Wärmeenergie von der Wärmequelle 12 aufnimmt und sich zumindest ein Abschnitt des Steuerelements 62 bei steigender Temperatur auf das Wärmeleitelement 16 zubewegt oder einen Druck auf das Wärmeleitelement 16 erhöht.

Bei dieser fünften Ausführungsform ist das Steuerelement 62 als ein erster Streifen 64 ausgebildet und weist zudem ein Gegenelement 66 auf, das als zweiter Streifen auf dem Steuerelement 62 fest angeordnet ist. Das Gegenelement 66 besteht aus einem Material, das einen höheren Wärmeausdehnungskoeffizienten hat als das Steuerelement 62. Das Steuerelement 62 ist mit dem Gegenelement 66 so angeordnet, dass das Steuerelement 62 bei steigender Temperatur mit steigendem Druck gegen das Wärmelement 16 drückt.

Es ist auch eine umgekehrte Anordnung möglich, bei der die Positionen von Steuerelement 62, also dem ersten Streifen 64, und dem Gegenelement 66 vertauscht sind. Das Gegenelement 66 besteht dann aus einem Material, das einen geringeren Wärmeausdehnungskoeffizienten hat als das Steuerelement 62, beispielsweise aus Carbon.

Fig. 8 zeigt die Situation bei der fünften Ausführungsform, wenn sich die Temperatur der Wärmequelle 12 erhöht hat, und das Steuerelement 62 das Wärmeleitelement 16 zusammengedrückt hat. Dabei ist bei dieser Ausführungsform das Wärmeleitelement 16 bevorzugt als elastische Wärmeleitpad ausgebildet, dessen Dicke sich bei steigendem Druck durch das Steuerelement 62 verringert.

Fig. 9 zeigt eine sechste Ausführungsform, bei der wiederum ein Steuerelement 62 mit einem Gegenelement 66, wie in Fig. 7 beschrieben, verwendet wird.

Bei der sechsten Ausführungsform weist das Wärmeleitelement 16 ein erstes kammartiges Element

68 und ein zweites kammartiges Kammelement 70 auf, die komplementär zueinander ausgebildet sind und miteinander kämmen. Das erste kammartige Element 68 ist am Steuerelement 62 angeordnet und das zweite kammartige Element 70 ist an der Wärmesenke 14 angeordnet. Das erste kammartige Element 68 und das zweite kammartige Element 70 schieben sich bei steigendem Druck durch das Steuerelement 62, also bei steigender Temperatur der Wärmequelle 12, weiter ineinander.

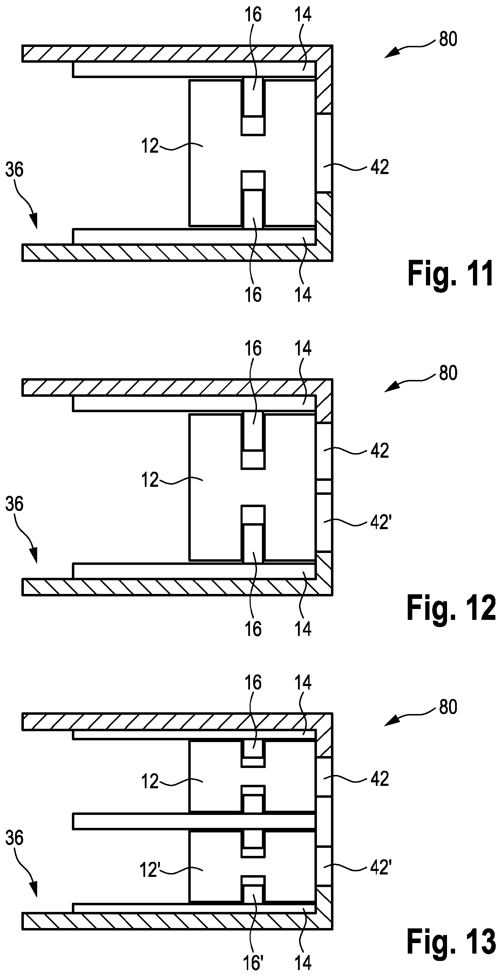

Fig. 11 zeigt eine erste Ausführungsform eines Videoendoskops 80 auf. In einem Gehäuse 36 ist hier eine Wärmequelle 12, die einen Bildsensor 42 aufweist, eine Wärmesenke 14 und ein Wärmeleitelement 16 angeordnet.

Fig. 12 zeigt eine zweite Ausführungsform eines Videoendoskops 80, wobei die Wärmequelle 12 einen ersten Bildsensor 42 und einen zweiten Bildsensor 42' aufweist.

Fig. 13 zeigt eine dritte Ausführungsform eines Videoendoskops 80, welches eine erste Wärmequelle 12 mit einem Bildsensor 42 und eine zweite Wärmequelle 12' mit einem zweiten Bildsensor 42' aufweist. Zusätzlich zu dem ersten Wärmeleitelement 16 ist hier auch ein zweites Wärmeleitelement 16' gezeigt.

**Patentansprüche**

1. Vorrichtung (10) zur Wärmeableitung mit einer Wärmequelle (12), einer Wärmesenke (14) und einem Wärmeleitelement (16), wobei das Wärmeleitelement entlang eines Wärmeleitwegs (18) Wärmeenergie (E) von der Wärmequelle zur Wärmesenke führt, und wobei das Wärmeleitelement (16) derart an der Wärmequelle (12) und der Wärmesenke (14) angeordnet ist und sich mit steigender Temperatur des Wärmeleitelements (16) derart physikalisch verändert, dass a) sich eine erste Querschnittsfläche (A) zwischen der Wärmequelle (12) und dem Wärmeleitelement (16) und/oder eine zweite Querschnittsfläche zwischen dem Wärmeleitelement (16) der Wärmesenke (14) vergrößert, und/oder b) sich die Länge (d) des Wärmeleitwegs (18) verkürzt.

2. Vorrichtung nach Anspruch 1, wobei das Wärmeleitelement (16) zur Steigerung der Wärmeableitung eine Heatpipe (26) aufweist oder von einem Fluid durchströmt wird.

3. Vorrichtung nach einem der vorhergehenden Ansprüche, wobei die Wärmequelle (12) eine erste Ausnehmung (20) aufweist, in der ein erster Abschnitt (22) des Wärmeleitelements (16) angeordnet ist, oder das Wärmeleitelement eine erste Ausnehmung aufweist, in der ein erster Abschnitt der Wärmequelle angeordnet ist.

4. Vorrichtung nach einem der vorhergehenden Ansprüche, wobei die Wärmesenke (14) eine zweite Ausnehmung (28) aufweist, in der ein zweiter Abschnitt (30) des Wärmeleitelements (16) angeordnet ist, oder das Wärmeleitelement (16) eine zweite Ausnehmung aufweist, in der ein zweiter Abschnitt der Wärmesenke (14) angeordnet ist.

5. Vorrichtung nach Anspruch 4, wobei die zweite Ausnehmung (28) durch die Wärmesenke (14) geführt ist und das Wärmeleitelement (16) in der zweiten Ausnehmung (28) durch die Wärmesenke (14) geführt ist.

6. Vorrichtung nach einem der vorhergehenden Ansprüche, wobei die Wärmequelle (12), die Wärmesenke (14) und das Wärmeleitelement (16) entlang einer Geraden angeordnet sind, insbesondere entlang einer gemeinsamen Längsmittelachse (34).

7. Vorrichtung nach einem der vorhergehenden Ansprüche, wobei die Wärmequelle (12), die Wärmesenke (14) und das Wärmeleitelement (16) innerhalb eines Gehäuses angeordnet sind, wobei eine dem Wärmeleitelement (16) abgewandte Seite (38) der Wärmequelle (12) und/oder eine dem Wärmeleitelement (16) abgewandte Seite (40) der Wärmesenke (14) am Gehäuse (36) angeordnet ist.

8. Vorrichtung nach Anspruch 7, wobei an der dem Wärmeleitelement (16) abgewandten Seite (38) der Wärmequelle (12) ein Bildsensor (42) ausgebildet ist, der eine Blickachse (44) aufweist, die durch eine Öffnung (46) in einer Wand (48) des Gehäuses (36) aus dem Gehäuse heraus gerichtet ist.

9. Vorrichtung nach einem der vorhergehenden Ansprüche, wobei die Vorrichtung (10) ferner ein Steuerelement (50) aufweist, das Wärmeenergie von der Wärmequelle (12) aufnimmt und bei steigender Temperatur einen steigenden Druck auf das Wärmeleitelement (16) ausübt.

10. Vorrichtung nach Anspruch 9, wobei die Vorrichtung (10) einen Hebel (52) mit einem ersten Hebelarm (54) und einem zweiten Hebelarm (56) aufweist, wobei das Steuerelement (50) bei steigender Temperatur einen steigenden Druck auf den ersten Hebelarm (54) ausübt, so dass der zweite Hebelarm (56) über die Wärmesenke (14) einen Druck auf das Wärmeleitelement (16) ausübt.

11. Vorrichtung nach einem der Ansprüche 1 bis 8, wobei die Vorrichtung (10) ferner ein Steuerelement (62) aufweist, das Wärmeenergie von der Wärmequelle (12) aufnimmt und sich zumindest ein Abschnitt des Steuerelements (62) bei steigender Temperatur auf das Wärmeleitelement (16) zubewegt oder einen Druck auf das Wärmeleitelement (16) erhöht.

12. Vorrichtung nach einem der Ansprüche 9 bis 11, wobei das Steuerelement (62) als ein erster Streifen (64) ausgebildet ist und ein Gegenelement (66) aufweist, das als zweiter Streifen auf dem Steuerelement (62) fest angeordnet ist, wobei das Gegenelement (66) aus einem Material besteht, das einen anderen Wärmeausdehnungskoeffizienten hat als das Steuerelement (62), wobei das Steuerelement (62) mit dem Gegenelement (66) so angeordnet ist, dass das Steuerelement (62) bei steigender Temperatur mit steigendem Druck gegen das Wärmeleitelement (16) drückt, und optional das Wärmeleitelement (16) als Wärmeleitpad ausgebildet ist, dessen Dicke sich bei steigendem Druck durch das Steuerelement (62) verringert.

13. Vorrichtung nach Anspruch 12 oder 13, wobei das Wärmeleitelement (16) ein erstes kammartiges Element (68) und ein zweites kammartiges Element (70) aufweist, die komplementär zueinander ausgebildet sind und miteinander kämmen, wobei das erste kammartige Element (68) am Steuerelement (62) angeordnet ist und das zweite kammartige Element (70) an der Wärmesenke (14) angeordnet ist, wobei sich das erste kammartige Element (68) und das zweite kammartige Element (70) bei steigendem Druck durch das Steuerelement (62) weiter ineinanderschieben.

14. Videoendoskop (80) mit einer Vorrichtung (10) nach einem der vorhergehenden Ansprüche, wobei die Wärmequelle (12) einen Bildsensor (42) aufweist.

15. Verwendung einer Vorrichtung (10) nach einem der vorhergehenden Ansprüche zur Wärmeableitung in einem Videoendoskop (80).

**Claims**

1. Apparatus (10) for heat dissipation, comprising a heat source (12), a heat sink (14) and a heat conducting element (16), wherein the heat conducting element guides heat energy (E) along a heat conducting path (18) from the heat source to the heat sink, and wherein the heat conducting element (16) is arranged on the heat source (12) and on the heat sink (14) in such a way, and changes physically as the temperature of the heat conducting element (16) increases in such a way that a) a first cross-sectional area (A) between the heat source (12) and the heat conducting element (16) and/or a second cross-sectional area between the heat conducting element (16) of the heat sink (14) increases, and/or b) the length (d) of the heat conducting path (18) becomes shorter.

2. Apparatus according to claim 1, wherein the heat conducting element (16) has a heat pipe (26) or is passed through by a fluid, in order to increase heat dissipation.

3. Apparatus according to either of the preceding claims, wherein the heat source (12) has a first recess (20) in which a first portion (22) of the heat conducting element (16) is arranged, or the heat conducting element has a first recess in which a first portion of the heat source is arranged.

4. Apparatus according to any of the preceding claims, wherein the heat sink (14) has a second recess (28) in which a second portion (30) of the heat conducting element (16) is arranged, or the heat conducting element (16) has a second recess in which a second portion of the heat sink (14) is arranged.

5. Apparatus according to claim 4, wherein the second recess (28) is guided through the heat sink (14) and the heat conducting element (16) is guided through the heat sink (14) in the second recess (28).

6. Apparatus according to any of the preceding claims, wherein the heat source (12), the heat sink (14) and the heat conducting element (16) are arranged in a straight line, in particular along a common longitudinal central axis (34).

7. Apparatus according to any of the preceding claims, wherein the heat source (12), the heat sink (14) and the heat conducting element (16) are arranged within a housing, wherein a face (38) of the heat source (12) facing away from the heat conducting element (16) and/or a face (40) of the heat sink (14) facing away from the heat conducting element (16) is arranged on the housing (36).

8. Apparatus according to claim 7, wherein an image sensor (42) is formed on the face (38) of the heat source (12) facing away from the heat conducting element (16), which image sensor has a visual axis (44) which is directed out of the housing through an opening (46) in a wall (48) of the housing (36).

9. Apparatus according to any of the preceding claims, wherein the apparatus (10) further has a control element (50) which absorbs heat energy from the heat source (12) and exerts increasing pressure on the heat conducting element (16) as the temperature increases.

10. Apparatus according to claim 9, wherein the apparatus (10) comprises a lever (52) having a first lever arm (54) and a second lever arm (56), wherein the control element (50) exerts increasing pressure on the first lever arm (54) as the temperature increases, so that the second lever arm (56) exerts pressure on the heat conducting element (16) by means of the heat sink (14).

11. Apparatus according to any of claims 1 to 8, wherein the apparatus (10) further comprises a control element (62) which absorbs heat energy from the heat source (12) and, as the temperature increases, at least a portion of the control element (62) moves toward the heat conducting element (16) or increases pressure on the heat conducting element (16).

12. Apparatus according to any of claims 9 to 11, wherein the control element (62) is designed as a first strip (64) and has a counterpart element (66) which is fixedly arranged as a second strip on the control element (62), wherein the counterpart element (66) is made of a material which has a different heat expansion coefficient than the control element (62), wherein the control element (62) is arranged together with the counterpart element (66) in such a way that the control element (62) presses against the heat conducting element (16) with increasing pressure as the temperature increases, and the heat conducting element (16) is optionally designed as a heat conducting pad, the thickness of which decreases as pressure exerted by the control element (62) increases.

13. Apparatus according to claim 12 or 13, wherein the heat conducting element (16) has a first comb-like element (68) and a second comb-like element (70) which are complementary to one another and mesh with one another, wherein the first comb-like element (68) is arranged on the control element (62) and the second comb-like element (70) is arranged on the heat sink (14), wherein the first comb-like element (68) and the second comb-like element (70) push further into one another as pressure from the control element (62) increases.

14. Video endoscope (80) comprising an apparatus (10) according to any of the preceding claims, wherein the heat source (12) has an image sensor (42).

15. Use of an apparatus (10) according to any of the preceding claims for heat dissipation in a video endoscope (80).

**Revendications**

1. Dispositif (10) pour la dissipation de chaleur comportant une source de chaleur (12), un puits de chaleur (14) et un élément conducteur de chaleur (16), dans lequel l'élément conducteur de chaleur guide de l'énergie thermique (E) le long d'un trajet de conduction de chaleur (18) depuis la source de chaleur jusqu'au puits de chaleur, et dans lequel l'élément conducteur de chaleur (16) est disposé sur la source de chaleur (12) et le puits de chaleur (14) et se modifie physiquement avec une augmentation de la température de l'élément conducteur de chaleur (16) de telle sorte que a) une première surface de section transversale (A) entre la source de chaleur (12) et l'élément conducteur de chaleur (16) et/ou une seconde surface de section transversale entre l'élément conducteur de chaleur (16) et le puits de chaleur (14) augmentent, et/ou b) la longueur (d) du trajet de conduction de chaleur (18) se raccourcit.

2. Dispositif selon la revendication 1, dans lequel, pour augmenter la dissipation de chaleur, l'élément conducteur de chaleur (16) présente un caloduc (26) ou est traversé par un fluide.

3. Dispositif selon l'une des revendications précédentes, dans lequel la source de chaleur (12) présente un premier évidement (20) dans lequel est disposée une première section (22) de l'élément conducteur de chaleur (16), ou l'élément conducteur de chaleur présente un premier évidement dans lequel est disposée une première section de la source de chaleur.

4. Dispositif selon l'une des revendications précédentes, dans lequel le puits de chaleur (14) présente un second évidement (28) dans lequel est disposée une seconde section (30) de l'élément conducteur de chaleur (16), ou l'élément conducteur de chaleur (16) présente un second évidement dans lequel est disposée une seconde section du puits de chaleur (14).

5. Dispositif selon la revendication 4, dans lequel le second évidement (28) est guidé à travers le puits de chaleur (14) et l'élément conducteur de chaleur (16) est guidé dans le second évidement (28) à travers le puits de chaleur (14).

6. Dispositif selon l'une des revendications précédentes, dans lequel la source de chaleur (12), le puits de chaleur (14) et l'élément conducteur de chaleur (16) sont disposés selon une droite, en particulier selon un axe médian longitudinal commun (34).

7. Dispositif selon l'une des revendications précédentes, dans lequel la source de chaleur (12), le puits de chaleur (14) et l'élément conducteur de chaleur (16) sont disposés à l'intérieur d'un boîtier, dans lequel un côté (38) de la source de chaleur (12) opposé à l'élément conducteur de chaleur (16) et/ou un côté (40) du puits de chaleur (14) opposé à l'élément conducteur de chaleur (16) sont disposés sur le boîtier (36).

8. Dispositif selon la revendication 7, dans lequel un capteur d'image (42) est réalisé sur le côté (38) de la source de chaleur (12) opposé à l'élément conducteur de chaleur (16), lequel capteur d'image présente un axe de vision (44) qui est dirigé vers l'extérieur du boîtier (36) à travers une ouverture (46) dans une paroi (48) du boîtier.

9. Dispositif selon l'une des revendications précédentes, dans lequel le dispositif (10) présente en outre un élément de commande (50) qui reçoit de l'énergie thermique provenant de la source de chaleur (12) et qui exerce une pression croissante sur l'élément conducteur de chaleur (16) lors de l'augmentation de la température.

10. Dispositif selon la revendication 9, dans lequel le dispositif (10) présente un levier (52) comportant un premier bras de levier (54) et un second bras de levier (56), dans lequel l'élément de commande (50) exerce une pression croissante sur le premier bras de levier (54) lors de l'augmentation de la température, de sorte que le second bras de levier (56) exerce une pression sur l'élément conducteur de chaleur (16) par l'intermédiaire du puits de chaleur (14).

11. Dispositif selon l'une des revendications 1 à 8, dans lequel le dispositif (10) présente en outre un élément de commande (62) qui reçoit de l'énergie thermique provenant de la source de chaleur (12) et au moins une section de l'élément de commande (62) se déplace vers l'élément conducteur de chaleur (16) ou augmente une pression sur l'élément conducteur de chaleur (16) lors de l'augmentation de la température.

12. Dispositif selon l'une des revendications 9 à 11, dans lequel l'élément de commande (62) est réalisé en tant que première bande (64) et présente un contre-élément (66) qui est disposé de manière fixe sur l'élément de commande (62) en tant que seconde bande, dans lequel le contre-élément (66) est constitué d'un matériau qui présente un coefficient de dilatation thermique différent de celui de l'élément de commande (62), dans lequel l'élément de commande (62) est disposé avec le contre-élément (66) de sorte que, lors de l'augmentation de la température, l'élément de commande (62) appuie avec une pression croissante contre l'élément conducteur de chaleur (16), et, éventuellement, l'élément conducteur de chaleur (16) est réalisé comme un coussinet conducteur de chaleur dont l'épaisseur diminue lorsque la pression exercée par l'élément de commande (62) augmente.

13. Dispositif selon la revendication 12 ou 13, dans lequel l'élément conducteur de chaleur (16) présente un premier élément en forme de peigne (68) et un second élément en forme de peigne (70) qui sont complémentaires l'un de l'autre et s'engrènent l'un dans l'autre, dans lequel le premier élément en forme de peigne (68) est disposé sur l'élément de commande (62) et le second élément en forme de peigne (70) est disposé sur le puits de chaleur (14), dans lequel le premier élément en forme de peigne (68) et le second élément en forme de peigne (70) continuent à s'engrener l'un dans l'autre lorsque la pression exercée par l'élément de commande (62) augmente.

14. Vidéoendoscope (80) comportant un dispositif (10) selon l'une des revendications précédentes, dans lequel la source de chaleur (12) présente un capteur d'image (42).

15. Utilisation d'un dispositif (10) selon l'une des revendications précédentes pour la dissipation de chaleur dans un vidéoendoscope (80).

**Fig. 1**

**Fig. 2**

**Fig. 3**

**Fig. 4**

**Fig. 5**

**Fig. 6**

Fig. 7

Fig. 8

Fig. 9

Fig. 10

Fig. 11

Fig. 12

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20170258309 A1

- US 3391728 A